# EUROPEAN PATENT APPLICATION

(11) **EP 1 541 111 A1**
(43) Date of publication of application: **15.06.2005**
(21) Application number: 03738625.7
(22) Date of filing: 01.07.2003
(51) Int. Cl.: A61H 33/14, A61G 10/00

(54) **CARBON DIOXIDE EXTERNAL ADMINISTRATION DEVICE**

(30) Priority: 01.07.2002 JP 2002192298
(71) Applicant: Neochemir Inc., Kobe-shi, Hyogo 651-0087 (JP)
(72) Inventor: TANAKA, Masaya, Kobe-shi, Hyogo 654-0036 (JP)
(74) Representative: Sajda, Wolf E., Dipl.-Phys.
(86) International application number: PCT/JP2003/008381
(87) International publication number: WO 2004/002393

(57) **Abstract**

The present invention provides a carbon dioxide external administration device according to which assured cosmetic and medical effects can be readily obtained. This administration device comprises a sealing enclosure member capable of sealing a body surface from the outside air, a supply means for supplying carbon dioxide into the inside of the sealing enclosure member, and an absorption aid that assists transdermal or transmucosal absorption of the carbon dioxide inside the sealing enclosure member. The administration device may further have a carbon dioxide amount indicator that expands upon carbon dioxide being supplied into the sealing enclosure member, and contracts by the decrease of carbon dioxide.

## Description

### TECHNICAL FIELD

The present invention relates to a carbon dioxide external administration device according to which cosmetic and medical effects are readily obtained.

### BACKGROUND ART

It is disclosed in Japanese Patent Application Laid-open No. 2000-319187 that compositions for transdermal or transmucosal absorption of carbon dioxide, that are viscous compositions containing carbon dioxide in the form of bubbles, are effective in general with conditions such as the following:
(1) itching accompanying mucocutaneous diseases or mucocutaneous disorders such as athlete's foot, insect bite, atopic dermatitis, nummular eczema, xeroderma, seborrheic eczema, urticaria, prurigo, housewives' eczema, acne vulgaris, impetigo, folliculitis, carbuncle, furunculosis, phlegmon, pyoderma, psoriasis, ichthyosis, palmoplantar keratoderma, lichen, pityriasis, wound, burn, rhagades, erosion and chilblain; mucocutaneous injuries such as decubitus ulcer, wound, burn, angular stomatitis, stomatitis, skin ulcer, rhagades, erosion, chilblain and gangrene ;
(2) incomplete taking of skin grafts, skin flaps etc.;
(3) dental diseases such as gingivitis, alveolar pyorrhea, denture ulcers, nigricans gingiva and stomatitis;
(4) skin ulcers, cryesthesia and numbness caused by peripheral circulatory disorders such as thromboangitis obliterans, arteriolosclerosis obliterans, diabetic peripheral circulatory disorders and lower limb varicosis;
(5) musculoskeletal diseases such as chronic rheumatoid arthritis, cervico-omo-brachial syndrome, myalgia, arthralgia and lumbago;
(6) nervous system diseases such as neuralgia, polyneuritis and subacute myelo-optic neuropathy;
(7) keratoses such as psoriasis, corns, calluses, ichthyosis, palmoplantar keratoderma, lichen and pityriasis;
(8) suppurative skin diseases such as acne vulgaris, impetigo, folliculitis, carbuncles, furuncles, phlegmon, pyoderma and suppurative eczema;
(9) suppression of hair re-growth after depilation (treatment of unwanted hair);
(10) cosmetic troubles with the skin or hair such as freckles, rough skin, loss of clarity of the skin, loss of elasticity or luster of the skin, and loss of glossiness of the hair, and partial obesity.

However, to obtain cosmetic or medical effects using such a conventional composition for transdermal or transmucosal absorption of carbon dioxide, the composition must be used in large quantities for a long time period, and considering this the effects are weak. For example, to obtain a face slimming effect using the composition, for example 26.2 g per day must be used for one month. Furthermore, such a composition has high viscosity, and hence is difficult to remove from the skin or mucosae after use, and thus use is bothersome.

On the other hand, a poultice for obtaining a blood circulation promoting effect using carbon dioxide gas (hereinafter used to be the same meaning as carbon dioxide) is proposed in Japanese Patent Application Laid-open No. 62-286922.

With this poultice, a cloth containing water is placed onto a cloth containing a carbonate and an organic acid to generate carbon dioxide gas, which is dissolved in the water contained in the cloth and is thus used as dissolved carbon dioxide gas; however, the reaction between a carbonate and an organic acid is generally very rapid, and hence the amount of carbon dioxide discharged into the atmosphere is greater than the amount dissolved in the water, and thus cosmetic or medical effects due to transdermal or transmucosal absorption of carbon dioxide cannot really be expected.

In view of this state of affairs, it is an object of the present invention to provide a carbon dioxide external administration device according to which cosmetic and medical effects are readily obtained.

### DISCLOSURE OF THE INVENTION

To attain the above object, the following technical means are adopted with the present invention.

That is, a carbon dioxide external administration device of the present invention is characterized by comprising a sealing enclosure member capable of sealing a body surface from the outside air, a supply means for supplying carbon dioxide into the inside of the sealing enclosure member, and an absorption aid that assists transdermal or transmucosal absorption of the carbon dioxide inside the sealing enclosure member.

With carbon dioxide, in a gaseous form, the efficiency of transdermal or transmucosal absorption is generally very low, but by applying a carbon dioxide absorption aid to skin or mucosae, and sealing the skin or mucosae and exposing the skin or mucosae to carbon dioxide for a certain time, the carbon dioxide is transdermally or transmucosally absorbed efficiently, and hence strong cosmetic and medical effects are obtained in a short period of time.

In the present invention, 'sealing enclosure member' means a member that is capable of sealing a body surface which is part or all of a human body, and is capable of holding therein a certain amount of a gas, with this gas not leaking out or leaking out only slightly.

In the present invention, there are no particular limitations on the carbon dioxide supply means, so long as this means can supply gaseous carbon dioxide into the inside of the sealing enclosure member; such supply means include, for example, ones that have carbon dioxide stored therein, with it being possible to release the carbon dioxide when required, such as a carbon dioxide gas cylinder, and ones that generate carbon dioxide when required through reaction between an acid and a carbonate.

In the present invention, carbon dioxide 'absorption aid' means a material that contains a carbon dioxide-dissolving medium such as water, an alcohol, oils and fats or a wax, and adheres to the skin or mucosae by being applied thereon, patched thereon or the like in accordance with the form, properties and so on of the absorption aid, thus forming a layer containing the carbon dioxide-dissolving medium on the skin or mucosae.

It is preferable for the carbon dioxide external administration device of the present invention to have a carbon dioxide amount indicator that expands upon carbon dioxide being supplied into the sealing enclosure member, and contracts by the decrease of carbon dioxide.

It is preferable for the sealing enclosure member to have the aforementioned carbon dioxide amount indicator, since then as the carbon dioxide filled into the sealing enclosure member is transdermally or transmucosally absorbed through the absorption aid, the carbon dioxide amount indicator, which was initially expanded, contracts, whereby it can easily be understood that the carbon dioxide is being transdermally or transmucosally absorbed even without specialized knowledge or the like. Furthermore, the aforementioned indicator has a simple structure and hence little failure or the like, and moreover can be made from a material such as a balloon and hence is inexpensive.

With the carbon dioxide external administration device of the present invention, it is preferable for the absorption aid to contain at least one carbon dioxide-dissolving medium selected from the group consisting of water, alcohols having a high vaporization temperature, and oils and fats.

Carbon dioxide dissolves in water, alcohols, oils and fats and waxes, and hence one or more of these can be used as a carbon dioxide-dissolving medium of the absorption aid. However, it is preferable for an alcohol to have a vaporization temperature as high as possible. An alcohol that vaporizes at the skin temperature of a human or a temperature lower than this is undesirable, since it will be difficult for the required amount to be retained on the skin or mucosae. Oils and fats may be liquid, or may be semi-solid as with a butter or the like, so long as the oil or the fat can be thinly spread over the skin or mucosae.

With the carbon dioxide external administration device of the present invention, it is preferable for the carbon dioxide absorption aid to be a sheet-like product impregnated with a liquid containing at least water.

If the carbon dioxide absorption aid is a sheet-like product impregnated with a liquid containing at least water, then the carbon dioxide absorption aid need merely be patched on the skin or mucosae in use and then peeled off after use, and hence it can be used very simply. Moreover, even if the amount of the liquid impregnated into the sheet-like product is low, repeated use is possible merely by replenishing the required amount of the liquid, and it is therefore very economical.

With the carbon dioxide external administration device of the present invention, it is preferable for the carbon dioxide absorption aid to contain a carbon dioxide-dissolving medium comprising a viscous material containing at least water.

When the carbon dioxide absorption aid contains a carbon dioxide-dissolving medium comprising a viscous material containing at least water, the carbon dioxide absorption aid can be applied even to a site that is not flat, and will not run off after having been applied onto the skin or mucosae, and will thus assist transdermal or transmucosal absorption of the carbon dioxide continually and reliably.

With the carbon dioxide external administration device of the present invention, it is preferable for the aforementioned carbon dioxide-dissolving medium comprising a viscous material containing at least water to contain at least one thickener selected from the group consisting of sodium alginate, propylene glycol alginate, carrageenan, sodium hyaluronate, pectin, polyvinyl alcohol and polyvinyl pyrrolidone.

The aforementioned thickeners will give the viscous material the required viscositywhen used in a small amount, have high affinity with skin and mucosae, and have good feeling in use, and hence can be suitably used with a cosmetic or medical purpose.

With the carbon dioxide external administration device of the present invention, it is preferable for the carbon dioxide-dissolving medium to comprise at least a calcium alginate hydrogel.

Calcium alginate forms a relatively tough hydrogel, and has high affinity with skin and mucosae and wound surfaces, protecting the same. Moreover, in the case that the carbon dioxide-dissolving medium of the carbon dioxide absorption aid is predominantly water and the form of the absorption aid is a hydrogel, there will be less evaporation of water from the surface thereof in comparison with a solution or the like, and hence there will be little decrease in the efficiency of dissolution of carbon dioxide into the carbon dioxide-dissolving medium, whereby cosmetic and medical effects can be readily obtained.

With the carbon dioxide external administration device of the present invention, it is preferable for the carbon dioxide-dissolving medium to comprise an emulsion or cream comprising at least an oil or a fat, a surfactant and water.

It is preferable for the carbon dioxide absorption aid to comprise an emulsion or cream comprising at least an oil or a fat, a surfactant and water, since the emulsion or cream readily penetrates into the stratum corneum, whereby not only is the carbon dioxide absorbed efficiently, but moreover cosmetic effects such as whitening and moisturizing are enhanced.

### BRIEF DESCRIPTION OF THE DRAWINGS

FIG. 1 is a schematic drawing of the constitution of a carbon dioxide external administration device according to the present invention.
FIG. 2 is a schematic drawing of the constitution of a carbon dioxide external administration device according to the present invention.
FIG. 3 is a schematic drawing of the constitution of a carbon dioxide external administration device according to the present invention.
FIG. 4 is a schematic drawing of the constitution of a carbon dioxide external administration device according to the present invention.
FIG. 5 is a perspective drawing of sealing enclosure members that can be used in a carbon dioxide external administration device according to the present invention.
FIG. 6 is a schematic drawing of the constitution of a carbon dioxide external administration device according to the present invention.
FIG. 7 is a schematic drawing of the constitution of a carbon dioxide external administration device according to the present invention.

### BEST MODE FOR CARRYING OUT THE INVENTION

Following is a description of embodiments of the present invention, with reference to the drawings.

A carbon dioxide external administration device 1 of the present invention is constituted from at least a sealing enclosure member 2 capable of sealing a body surface from the outside air, a supply means 3 for supplying carbon dioxide into the inside of the sealing enclosure member 2, and an absorption aid 4 that assists transdermal or transmucosal absorption of the carbon dioxide inside the sealing enclosure member 2. Note, however, that the sealing enclosure member 2 does not necessarily have to seal the body surface from the outside air completely, but rather it is sufficient so long as the carbon dioxide concentration inside the sealing enclosure member 2 can be maintained above a certain level by continually supplying a small amount of carbon dioxide into the sealing enclosure member 2.

With the carbon dioxide external administration device 1 of the present invention, there are no particular limitations on the shape, materials and so on of the sealing enclosure member 2, so long as the sealing enclosure member 2 covers the skin or mucosae and has therein a certain space in which carbon dioxide is held.

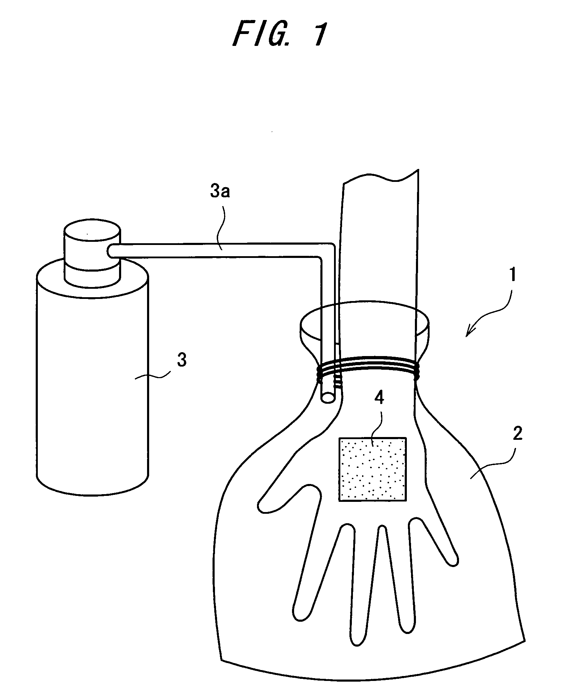

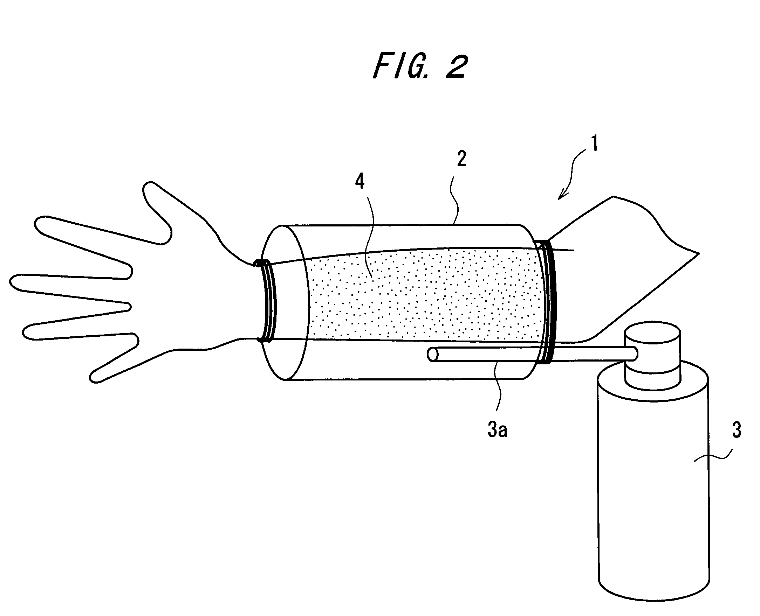

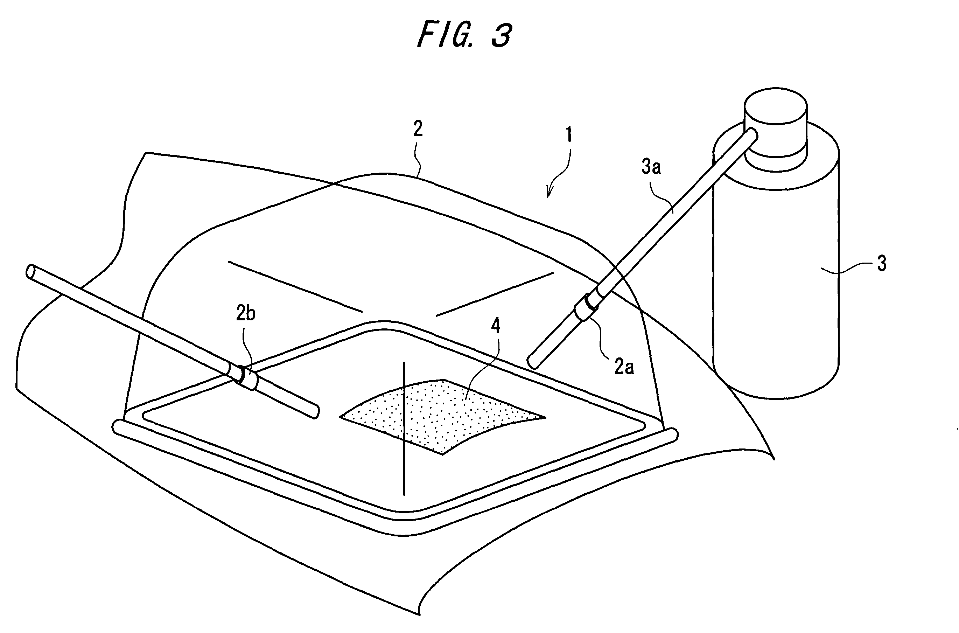

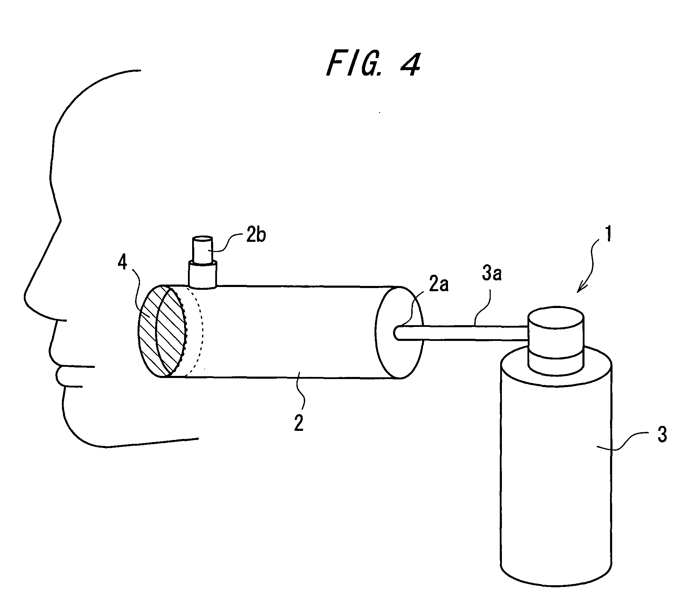

Regarding the shape, for example a bag that can completely pack a hand (or a foot) as shown in FIG. 1, and a tubular bag that wraps around an arm (or a leg) as shown in FIG. 2 can be used. Moreover, as the shape of the sealing enclosure member 2, a vessel having an open hemline that adheres to a relatively large area of the skin surface such as the abdomen or the like as shown in FIG. 3, or a cup-shaped tubular body that has one end thereof open and the other end thereof made to be a part connecting to the supply means 3 as shown in FIG. 4 can be used. Of course, the shape of the sealing enclosure member 2 is not limited to these examples.

There are no particular limitations on the materials of the sealing enclosure member 2 so long as these materials are impermeable to gases; materials can be selected from metal, plastic, rubber, glass and so on and used as appropriate in accordance with the purpose, the site of use and so on.

In the case that the sealing enclosure member 2 is one made of only a non-elastic and hard material such as metal or glass (hereinafter referred to as a 'rigid sealing enclosure member'), a shape having therein a certain sealed space surrounding the site of use is preferable. The part contacting with the skin or mucosae preferably has a shape conforming to the site of use so that carbon dioxide will not leak out; combining with an elastic material such as rubber or a resin for the part contacting with the skin or mucosae is preferable since it will be possible to flexibly respond to the site of use. The combination with a material that has higher flexibility and excellent adhesion to skin or mucosae such as a viscoelastic gel is more preferable.

Moreover, to fill carbon dioxide into the sealed space in which the skin or mucosae is sealed by the rigid sealing enclosure member, the enclosure member preferably has a gas outlet therein. When injecting carbon dioxide into the sealed space, air in the sealed space escapes out from a gap in the part contacting with the skin or mucosae and the air is replaced with carbon dioxide. But the adjustment of the size of the gap is so hard that it is difficult to carry out the replacement reliably, and the amount of carbon dioxide required for the replacement isn't fixed. For example, by providing a gas outlet in, a position furthest from the gas inlet, the replacement of the air with carbon dioxide can be carried out efficiently in a short period of time.

The gas inlet 2a in the rigid sealing enclosure member (see FIGS. 3 and 4) has connected thereto a tube 3a that is connected to the supply means 3; there are no particular limitations on the gas inlet 2a so long as gas will not leak therefrom, but one equipped with a check valve for preventing back flow of gas is more preferable. Regarding the gas outlet 2b in the rigid sealing enclosure member (see FIGS. 3 and 4), a gap in the part contacting with the skin or mucosae may be used, but to carry out the replacement of the air inside the enclosure member with carbon dioxide reliably and efficiently, a gas outlet equipped with a check valve for preventing back flow of gas is more preferable.

In the case that the sealing enclosure member 2 is one made of a material that is a flexible material such as a rubber, a resin or a soft plastic, especially, a material that has a shape holding ability, by which the shape is barely kept by some external force (hereinafter referred to as a 'flexible sealing enclosure member'), the part contacting with the skin or mucosae is also flexible, and hence the above material can be used as is. However, the combination with a material that has yet higher flexibility and excellent adhesion to skin or mucosae such as a viscoelastic gel for the part contacting with the skin or mucosae is more preferable. Moreover, with such a flexible sealing enclosure member, the same gas inlet and gas outlet as those in the case of a rigid sealing enclosure member can be used.

In the case that the sealing enclosure member 2 is one made of a material that is a flexible material such as a rubber or a resin, especially a material that is elastic highly like a balloon (hereinafter referred to as an 'elastic sealing enclosure member'), this can be used in the form of a tube, a bag or the like, with the skin or mucosae being sealed by binding the mouth of the tube, bag or the like covering the desired site, or providing a retractable opening/closing mouth in which rubber, a spring or the like is placed in the mouth.

Moreover, in the case of a tubular elastic sealing enclosure member for which the site of application is made to be, for example, an arm, the circumference of the tube may be made to be approximately the same as that of the arm, or may be made smaller, with the skin or mucosae being sealed in a state with the sealing enclosure member adhering to the skin or mucosae, or a state with only a very small space between the sealing enclosure member and the skin or mucosae; carbon dioxide may then be injected between the sealing enclosure member and the skin or mucosae so as to expand the sealing enclosure member, and the certain space in which carbon dioxide may be held is created by the carbon dioxide itself.

A gas inlet and gas outlet of the elastic sealing enclosure member may have the same structure as with the aforementioned rigid sealing enclosure member, but alternatively it is possible to seal the skin or mucosae with a tube, a bag or the like, and discharge the air therein as much as possible in advance, and then insert the tube 3a in from the orifice of the elastic sealing enclosure member having the form of a tube, a bag or the like such that air is prevented from entering therein as much as possible, and inject carbon dioxide (see FIGS. 1 and 2).

In the case that the sealing enclosure member 2 is one made of a foldable sheet- or film-like material (hereinafter referred to as a 'sheet-type sealing enclosure member'), this can be used in the form of a tube, a bag or the like, with the skin or mucosae being sealed by binding the orifice of the tube, the bag or the like covering the desired site, or providing a retractable opening/closing orifice in which rubber, a spring or the like is placed in the orifice.

Moreover, in the case of a tubular sheet-type sealing enclosure member for which the site of application is made to be, for example, an arm (or a leg), it is possible to make the circumference of the tube be greater than that of the arm, seal the arm and fold the sealing enclosure member, so as to discharge air therein as much as possible in advance, and then use the sealing enclosure member with adhering to the skin or mucosae. A gas inlet and gas outlet of the sealing enclosure member may be as with the rigid sealing enclosure member, but alternatively it is possible to seal the skin or mucosae with the tube, bag or the like, and discharge the air therein as much as possible in advance, and then insert a tube in from the orifice of the sheet-type sealing enclosure member having the form of a tube, a bag or the like such that air is prevented from entering therein as much as possible, and inject carbon dioxide.

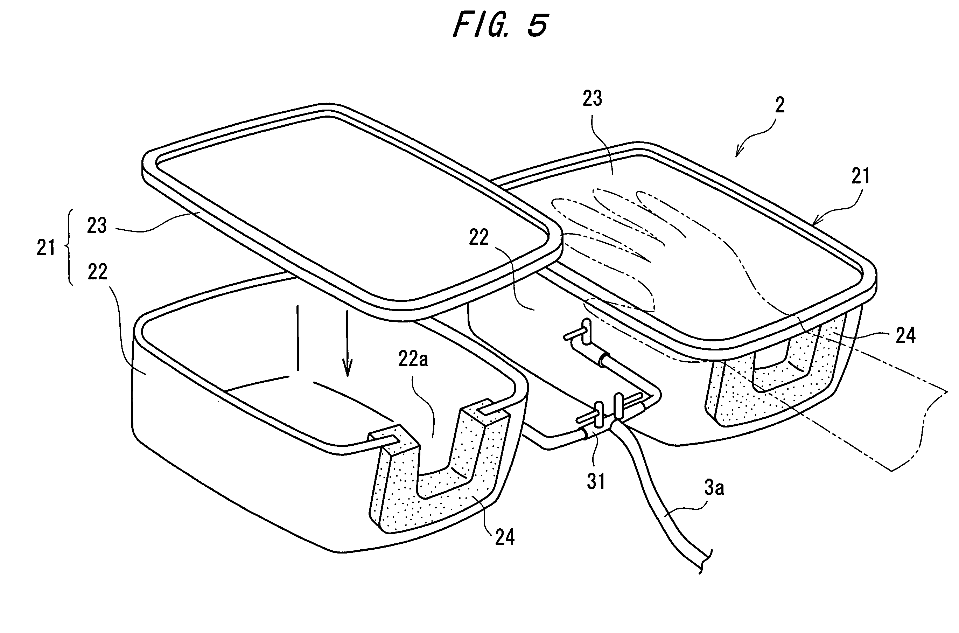

FIG. 5 shows an example of sealing enclosure members 2 that can be suitably used in the present invention.

These sealing enclosure members 2 comprise a right and left pair of storage boxes 21 made of a transparent or translucent synthetic resin; a tube 3a possessing a valve 31 is connected to the storage boxes 21, whereby carbon dioxide from the supply means 3 can be supplied to either or both of the storage boxes 21.

Each storage box 21 comprises a box body 22 that is open at the top and in a side wall thereof a cut-out concave portion 22a, through which a wrist can be inserted, is formed, and a detachable lid member 23 that covers the upper opening of the box body 22; a sealing member 24 made of a flexible material such as sponge is attached around the rim of the cut-out concave portion 22a. Moreover, although not shown in FIG. 5, a sealing member made of a flexible material such as sponge is also attached to the place corresponding to the cut-out concave portion 22a of the rim of the rear surface of the lid member 23.

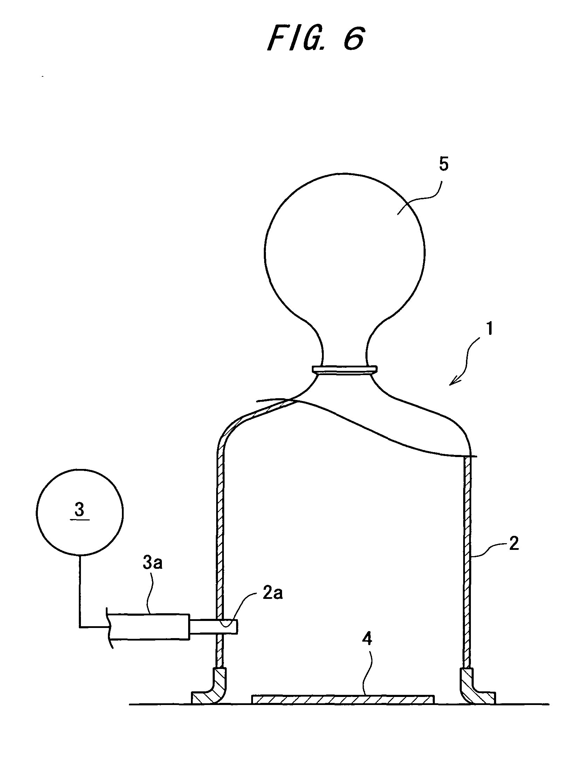

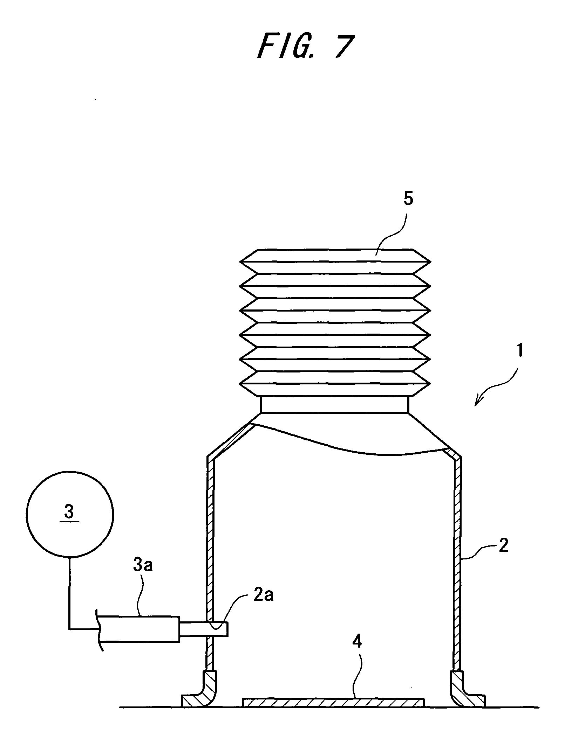

FIGs. 6 and 7 show carbon dioxide external administration devices 1 according to other embodiments of the present invention.

With each of the administration devices 1 in this case, a carbon dioxide amount indicator 5 that expands upon carbon dioxide being supplied into the sealing enclosure member 2 and contracts by the decrease of carbon dioxide is attached to an upper end of the sealing enclosure member 2, whereby transdermal or transmucosal absorption of carbon dioxide can easily be checked upon from the outside even without specialized knowledge or the like.

Moreover, of the administration devices 1 shown in FIGS. 6 and 7, with the administration device 1 shown in FIG. 6, a balloon indicator 5 made of rubber or another elastic material is used, and with the administration device 1 shown in FIG. 7, a bellows-structured indicator 5 that is retractable in a vertical direction is used.

With the carbon dioxide external administration device 1 of the present invention, there are no particular limitations on the carbon dioxide supply means 3; for example, a commercially available carbon dioxide gas cylinder or the like can be used. Moreover, there may be used, for example, a device that uses a closed vessel having a tube attached thereto, wherein so-called dry ice, which is solid carbon dioxide, is vaporized inside the vessel, or carbon dioxide is generated inside the vessel through reaction between a carbonate and an acid, and the like.

With the carbon dioxide external administration device 1 of the present invention, regarding the tube 3a connecting the sealing enclosure member 2 to the carbon dioxide supply means 3, there are no particular limitations so long as the tube is such that gas will not leak out, and any material from which a tube can be formed, for example, rubber, a resin, metal or glass, may be used.

With the carbon dioxide external administration device 1 of the present invention, there are no particular limitations on a sheet-like product used for the carbon dioxide absorption aid 4, so long as this sheet-like product can be impregnated with a liquid containing at least water and can be patched on skin or mucosae; examples thereof include woven and nonwoven cloths made of natural fibers, synthetic fibers, semi-synthetic fibers or the like, semi-permeable membranes such as cellulose membranes, and hydrogel sheets made of a natural polymer, a synthetic polymer, a semi-synthetic polymer or the like, and one or more of these may be used.

There are no particular limitations on the aforementioned liquid containing at least water; this liquid may be water itself, or so long as the effects of the present invention are not impaired, may be water having some substances dissolved or dispersed therein.

Moreover, the liquid containing at least water preferably has a pH of 7 to 2, and more preferably is acidic water having a pH of 6.5 to 4. The reason is that when carbon dioxide is dissolved in an acidic solvent of pH of not less than 4, the carbon dioxide is transdermally or transmucosally absorbed efficiently.

With the carbon dioxide external administration device 1 of the present invention, a viscous material used for the carbon dioxide absorption aid 4 may be liquid or semi-solid, so long as the viscous material contains water and does not easily run off or after having been applied onto the skin or mucosae, and has a viscosity of not less than 20 cps at 20°C; examples of forms include liquids, creams, pastes and gels; and one or more of these may be used.

Moreover, the viscous material preferably has a pH of 7 to 2, and more preferably is an acidic viscous material having a pH of 6.5 to 4.

There are no particular limitations on the viscous material, but the water content is preferably as high as possible, and moreover it is preferable to be one that is able to replenish moisture in the skin or mucosae. As a liquid, an aqueous solution, suspension, swelling liquid or the like of a thickener may be suitably used, because the required viscosity can be obtained with a small amount, and adhesion or viscosity to skin or mucosae is excellent. There are no particular limitations on the production method thereof, with it being possible to use a known method.

There are no particular limitations on a cream, a paste or a gel; one produced using a known method can be used.

As the thickener used in the viscous material, at least one selected from the group consisting of natural polymers, semi-synthetic polymers, synthetic polymers and inorganic materials can be used.

Examples of natural polymers include plant-derived polymers such as gum arabic, carrageenan, galactan, agar, xanthan gum, quince seed gum, guar gum, tragacanth, pectin, mannan, locust bean gum, wheat starch, rice starch, corn starch and potato starch, microorganism-derived polymers such as curdlan, xanthan gum, succinoglucan, dextran, hyaluronic acid and pullulan, and protein-type polymers such as albumin, casein, collagen, gelatin and fibroin; one or more of these can be used. Of these, from the standpoint of affinity with skin and mucosae and so on, gum arabic, carrageenan, xanthan gum, tragacanth, hyaluronic acid, pullulan, pectin, mannan and locust bean gum can be preferably used, and from the standpoint of feeling in use and so on, carrageenan and pectin can be more preferably used.

Examples of semi-synthetic polymers include cellulosic polymers such as ethylcellulose, carboxymethylcellulose, carboxymethylethylcellulose, carboxymethyl starch, croscarmellose, crystalline cellulose, cellulose acetate, cellulose acetate phthalate, hydroxyethylcellulose, hydroxypropylcellulose, hydroxypropylmethylcellulose, hydroxypropylmethylcellulose phthalate, methylcellulose and methylhydroxypropylcellulose, starch-type polymers such as pregelatinized starch, partially pregelatinized starch, carboxymethyl starch, dextrin, methyl starch, starch-acrylic acid copolymers and cellulose-acrylonitrile graft copolymers, alginate-type polymers such as sodium alginate and propylene glycol alginate, and other polysaccharide-type polymers such as chondroitin sulfate sodium and sodium hyaluronate; one or more of these can be used.

Of these, from the standpoint of affinity with skin and mucosae and so on, sodium alginate, propylene glycol alginate, sodium carboxymethylcellulose, dextrin and sodium hyaluronate can be preferably used, and from the standpoint of feeling in use and so on, sodium alginate, propylene glycol alginate and sodium hyaluronate can be more preferably used.

Examples of synthetic polymers include carboxyvinyl polymers, sodium polyacrylate, polyamines, polyacrylamide, polyvinylacetal diethylaminoacetate, polyvinyl alcohol, polyvinyl pyrrolidone, polyethylene glycol, polyethylene oxide, poly(meth)acrylic acid, polyvinyl methyl ether and so on; one or more of these can be used. Of these, from the standpoint of affinity with skin and mucosae and so on, carboxyvinyl polymers, sodium polyacrylate, polyacrylamide, polyvinyl alcohol and polyvinyl pyrrolidone can be preferably used, and from the standpoint of feeling in use and so on, polyvinyl alcohol, polyvinyl pyrrolidone and carboxyvinyl polymers can be more preferably used.

Examples of inorganic materials include hydrated silicon dioxide, light anhydrous silicic acid, colloidal alumina, bentonite, laponite and so on; one or more of these can be used.
With the carbon dioxide external administration device 1 of the present invention, there are no particular limitations on an alcohol having a high vaporization temperature used in the carbon dioxide absorption aid 4, so long as this alcohol is liquid or semi-solid at room temperature and does not readily vaporize at the skin temperature of a human; examples thereof include monohydric alcohols such as isopropyl alcohol and 1-butanol, and polyhydric alcohols such as ethylene glycol, diethylene glycol, polyethylene glycol, propylene glycol, dipropylene glycol, 1,3-butanediol, 1,2-pentanediol, isoprene glycol, glycerol, diglycerol, triglycerol and tetraglycerol; one or more of these can be used. There are no particular limitations on the method of using the alcohol having a high vaporization temperature; in accordance with the physical properties thereof and so on, the alcohol may be applied or sprayed onto the skin or mucosae as is, or impregnated or the like into a nonwoven cloth or the like and then patched on.

With the carbon dioxide external administration device 1 of the present invention, there are no particular limitations on an oil or a fat used in the carbon dioxide absorption aid 4, so long as this oil or fat is liquid or semi-solid at room temperature and can be applied relatively thinly onto the skin or mucosae; examples thereof include natural vegetable oils and fats such as avocado oil, avocado butter, olive oil, sesame oil, safflower oil, soybean oil, camellia oil, sunflower oil and macadamia nut oil, and animal oils and fats such as squalane, mink oil, beef tallow, lard, chicken fat and horse fat; one or more of these can be used.

There are no particular limitations on the method of using an oil or a fat; in accordance with the physical properties thereof and so on, the oil or the fat may be applied or sprayed onto the skin or mucosae as is, or impregnated or the like into a nonwoven cloth or the like and then patched on.

With the carbon dioxide external administration device 1 of the present invention, there are no particular limitations on a wax used in the carbon dioxide absorption aid 4, so long as this wax is liquid or semi-solid at room temperature and can be applied relatively thinly onto the skin or mucosae; examples thereof include jojoba oil, carnauba wax, candelilla wax, beeswax and lanolin; one or more of these can be used.

There are no particular limitations on the method of using the wax; in accordance with the physical properties thereof and so on, the wax may be applied or sprayed onto the skin or mucosae as is, or impregnated or the like into a nonwoven cloth or the like and then patched on.

With the carbon dioxide external administration device 1 of the present invention, when the carbon dioxide absorption aid 4 is applied to the skin or mucosae, it is preferable to form a film of the carbon dioxide-dissolving medium on the skin or mucosae as thin as possible. When the film of the carbon dioxide-dissolving medium is too thick, it takes time for the carbon dioxide to dissolve in the medium, and to diffuse and be absorbed into the skin or mucosae, and hence the carbon dioxide absorption efficiency may be poor. However, in the case that the vaporization temperature of the carbon dioxide-dissolving medium is relatively low, when the film of the carbon dioxide-dissolving medium formed is too thin, the medium may vaporize or evaporate due to the skin temperature and hence may be lost from the skin or mucosae while the carbon dioxide is being absorbed; the amount of the carbon dioxide-dissolving medium to be used must thus be suitably adjusted.

Raw materials commonly used in external preparations and cosmetics, for example fragrances, colorants, surfactants, oils, moisturizers, alcohols, preservatives, antioxidants, sequestering agents, anti-colorants, ultraviolet absorbing/scattering agents, vitamins, amino acids, drugs such as arbutin, koj ic acid, nutrients, anti-inflammatories, vasodilators, hormones, astringents, antihistamines, microbicides, sebum inhibiting agents, keratin stripping/dissolving agents, anti-seborrheic agents and antipruritics, and so on, may be blended into the carbon dioxide absorption aid 4, whereby the carbon dioxide absorption aid 4 can be used yet more suitably for a cosmetic or medical purpose.

With the carbon dioxide external administration device 1 of the present invention, the carbon dioxide used is gaseous, and the proportion of carbon dioxide in the gas is preferably not less than 10%, more preferably not less than 30%. The amount of carbon dioxide to be administered is preferably not less than 0.1 mg, more preferably not less than 0.3 mg, per square centimeter of the skin or mucosae.

Following is a more concrete description of the present invention giving examples; however, the present invention is not limited to these examples.

### [Example 1]

### (Sealing enclosure member 2)

As shown in FIG. 1, the end of a 15 cm x 30 cm transparent polyethylene bag was bound with a rubber band, thus producing a sealing enclosure member.

### (Carbon dioxide supply means 3)

A commercially available carbon dioxide gas cylinder for aquatic plant cultivation (trade name Tetra CO2 gas cylinder, made by Warner Lambert) was used.

### (Carbon dioxide absorption aid 4)

A carbon dioxide absorption aid 4 was produced by soaking a 0.5% aqueous citric acid solution into a polypropylene nonwoven cloth to an extent such that the water did not drip out.

The absorption aid 4 was cut into a 5 cm × 5 cm square, and was patched onto the skin of the back of the left hand of a 46-year-old male, and the left hand was covered with the sealing enclosure member 2, and the air therein was removed. A vinyl tube of outside diameter 6. 0 mm, inside diameter 3. 5 mm and length 60 cm having one end thereof connected to the carbon dioxide jet of the carbon dioxide supply means 3 was inserted into the sealing enclosure member 2. The mouth of the sealing enclosure member 2 was bound with a rubber band at the wrist part and thus sealed. 100 ml of carbon dioxide was filled into the sealing enclosure member 2 from the carbon dioxide supply means 3, and then the system was left for 5 minutes.

After 5 minutes, the left hand was removed from the sealing enclosure member 2, whereupon the skin under the carbon dioxide absorption aid 4 had reddened in a square shape matching that of the absorption aid 4. After 2 minutes, the reddening disappeared, and the square of skin turned very white, and a moistness was felt.

The water on the site on which the carbon dioxide absorption aid 4 had been patched was completely wiped off, and after 10 minutes the skin moisture content and sebum content were measured using a facial analyzer (made by Corefront Corporation; measures the skin moisture content and the sebum content in a range of 0 to 99).

The skin onto which the carbon dioxide absorption aid 4 of the present example had been applied had a skin moisture content of 71 and a sebum content of 48, which were clearly increased compared with a skin moisture content of 19 and a sebum content of 4 for the back of the untreated right hand, correlating to the feeling of moistness.

The skin surface was observed at a magnification of 40× using a CCD camera, whereupon at the site of application of the carbon dioxide absorption aid of the present example, compared with the skin on the back of the untreated right hand, the skin grooves and mounds were clearer, and the skin texture had become finer.

### [Example 2]

### (Sealing enclosure member 2)

As shown in FIG. 2, both ends of a transparent tubular polyethylene bag having a circumference of 30 cm and a length of 20 cm were bound with elastic cord, thus producing a sealing enclosure member.

### (Carbon dioxide supply means 3)

A commercially available carbon dioxide gas cylinder for aquatic plant cultivation (trade name Tetra CO2 gas cylinder, made by Warner Lambert) was used.

### (Carbon dioxide absorption aid 4)

A viscous material was prepared using 1.8 parts by weight of sodium alginate and 1 part by weight of sodium carboxymethyl cellulose as thickeners, 0.2 part by weight of methylparaben as a preservative, and 97 parts by weight of purified water as water, and this viscous material was used as a carbon dioxide absorption aid 4.

1 g of the aforementioned absorption aid 4 was applied onto the whole of the right upper arm of a 26-year-old female, and this upper arm was covered with the sealing enclosure member 2, and the air therein was removed. A vinyl tube of outside diameter 6.0 mm, inside diameter 3.5 mm and length 60 cm having one end thereof connected to the carbon dioxide jet of the carbon dioxide supply means 3 was inserted into the sealing enclosure member 2. Both ends of the sealing enclosure member 2 were bound with elastic cord and thus sealed. 100 ml of carbon dioxide was filled into the sealing enclosure member from the carbon dioxide supply means 3, and then the system was left for 10 minutes.

After 10 minutes, the upper arm was removed from the sealing enclosure member 2. The skin onto which the carbon dioxide absorption aid 4 had been applied had reddened, and hence an increase in blood flow was observed. The absorption aid was immediately completely removed, whereupon after 2 minutes the reddening disappeared, and the skin turned very white, and a moistness was felt. The circumference of the central part of the upper arm was measured, whereupon this circumference had decreased by 1.5 cm compared with that before the application of the carbon dioxide absorption aid 4.

Furthermore, after 10 minutes the skin moisture content and sebum content were measured using the aforementioned facial analyzer. The site onto which the carbon dioxide absorption aid 4 of the present example had been applied had a skin moisture content of 67 and a sebum content of 44, which were clearly increased compared with a skin moisture content of 11 and a sebum content of 3 for the untreated left upper arm, correlating to the feeling of moistness.

### [Example 3]

### (Sealing enclosure member 2)

A plastic box-shaped closed vessel as shown in FIG. 3 (length 10 cm × width 4 cm × depth 3 cm, having suction rubber at the part thereof contacting the human body, and a check valve for discharging gas from inside the vessel in the side thereof) was taken as a sealing enclosure member.

### (Carbon dioxide supply means 3)

A commercially available carbon dioxide gas cylinder for aquatic plant cultivation (trade name Tetra CO2 gas cylinder, made by Warner Lambert) was used.

### (Carbon dioxide absorption aid 4)

A viscous material was prepared using 2 parts by weight of carrageenan and 1 part by weight of pectin as thickeners, 0.2 part by weight of methylparaben as a preservative, and 96.8 parts by weight of purified water as water, and this viscous material was used as a carbon dioxide absorption aid 4.

A suitable amount of the aforementioned viscous material was thinly applied onto itchy atopic dermatitis that had arisen on the right thigh of a 13-year-old male (length approximately 5 cm × width approximately 3 cm). The aforementioned plastic box-shaped closed vessel, a check valve-possessing gas inlet of which had been connected to the carbon dioxide jet of the carbon dioxide gas cylinder by a vinyl tube of outside diameter 6.0 mm, inside diameter 3.5 mm and length 60 cm, was placed over the site in question, thus sealed the site.

150 ml of carbon dioxide was blown into the vessel from the gas cylinder, thus driving out the air in the vessel from the check valve not having the vinyl tube connected thereto, and then the system was left for 10 minutes. 1 minute after the carbon dioxide had been blown in, the atopic dermatitis turned red, and hence an increase in blood flow was observed. After 10 minutes, the viscous material was completely removed, whereupon the redness quickly disappeared; the dryness of the atopic dermatitis was eliminated, and the itchiness also went away.

### [Example 4]

### (Sealing enclosure member 2)

A plastic tubular body as shown in FIG. 4 was taken as a sealing enclosure member. This tubular body had a diameter of 3 cm and a length of 12 cm, had a check valve for discharging gas from inside the tube provided close to the tip of the tube, and had a carbon dioxide absorption aid 4 attached to the tip of the tube.

### (Carbon dioxide supply means 3)

A commercially available carbon dioxide gas cylinder for aquatic plant cultivation (trade name Tetra CO2 gas cylinder, made by Warner Lambert) was used.

### (Carbon dioxide absorption aid 4)

A viscous liquid prepared using 2 parts by weight of sodium alginate as a thickener and 98 parts by weight of purified water as water was soaked into a polyester nonwoven cloth of diameter 4 cm, and then the nonwoven cloth was immersed in a 1% aqueous calcium chloride solution, thus preparing a calcium alginate hydrogel membrane having a nonwoven cloth as a support; this hydrogel membrane was used as the carbon dioxide absorption aid 4.

The carbon dioxide absorption aid 4 was placed against the tip of the sealing enclosure member 2, folded around the sealing enclosure member 2, and fixed with a thick rubber band. The absorption aid 4 was then placed against the pigmented spot of diameter approximately 1 cm on the left cheek of a 42-year-old female, approximately 200 ml of carbon dioxide was injected in from the carbon dioxide supply means 3, thus replacing the air inside the sealing enclosure member 2 with carbon dioxide, and then the system was left for 10 minutes. This was continued once per day for 2 weeks, whereupon the pigmented spot became fainter.

### [Example 5]

### (Sealing enclosure member 2)

A sealing enclosure member 2 as shown in FIG. 5 was used.

More specifically, this sealing enclosure member was constituted from a polypropylene main vessel (length 20 cm × width 15 cm × depth 8 cm, having a three-way stopcock attached to a side thereof, having a cut of width 8 cm × depth 6 cm in an edge thereof, and having in the cut a U-shaped sponge of thickness 4 cm × width 10 cm × height 7 cm having a 5 cm × 5 cm cut-out therein, the sponge being fitted into the cut through the sponge having a 1 cm-deep groove formed therein along a centerline of the outside thereof), and a polyethylene lid having a sponge of width and height 1 cm and length 10 cm glued by double-sided tape to a part of the lid that contacts the top of the cut in the main vessel.

### (Carbon dioxide supply means 3)

Liquefied carbon dioxide gas filled into a high-pressure gas cylinder (filling amount: 30 kg) was used.

### (Carbon dioxide absorption aid 4)

A carbon dioxide absorption aid 4 comprising a viscous material containing water was prepared using 2.5 parts by weight of sodium alginate and 1.2 parts by weight of sodium carboxymethyl cellulose as thickeners, 88.2 parts by weight of purified water as water, 0.1 part by weight of methylparaben as a preservative, and 1 part by weight of sodium dihydrogen phosphate as a pH adjuster.

A suitable amount of the aforementioned carbon dioxide absorption aid 4 was applied onto the abrasion of diameter 5 mm on the back of the left hand of a 37-year-old female, the left hand was put into the aforementioned sealing enclosure member 2, and carbon dioxide was initially supplied into the sealing enclosure member 2 for 10 seconds at a rate of 200 mL/s from the aforementioned carbon dioxide supply means 3, and then for 5 minutes at a rate of 20 mL/s. The aid was then washed off, whereupon the pain previously felt upon contact with water had completely gone, and hence a wound healing promoting effect was observed.

### [Example 6]

### (Sealing enclosure members 2)

Sealing enclosure members 2 as shown in FIG. 5 were used.

### (Carbon dioxide supply means 3)

Liquefied carbon dioxide gas filled into a high-pressure gas cylinder (filling amount: 30 kg) was used.

### (Carbon dioxide absorption aid 4)

The same carbon dioxide absorption aid 4 as in Example 5 was spread to a thickness of approximately 0.5 mm onto a flat plastic plate, and a 5% calcium chloride solution was poured on from above so as to convert the sodium alginate into a hydrogel, thus preparing a carbon dioxide absorption aid 4 comprising a calcium alginate hydrogel on one side and a sodium alginate sol on the other side.

A 5 cm × 5 cm square of the carbon dioxide absorption aid 4 was patched onto the back of the left hand of a 48-year-old male, and with nothing patched onto the back of the right hand, the two hands were put respectively into the two sealing enclosure members 2a and 2b, and carbon dioxide was initially supplied into the sealing enclosure members 2 for 10 seconds at a rate of 200 mL/s from the carbon dioxide supply means 3, and then for 7 minutes at a rate of 20 mL/s.

Both hands of the male immediately became warm, but the back of the left hand felt warmer than the back of the right hand. The absorption aid was subsequently removed, whereupon the skin on the back of the left hand had become smooth and white, and hence whitening and skin beautification effects were observed.

### [Examples 7 to 11]

### (Sealing enclosure members 2)

Sealing enclosure members 2 as shown in FIG. 5 were used.

### (Carbon dioxide supply means 3)

Liquefied carbon dioxide gas filled into a high-pressure gas cylinder (filling amount: 30 kg) was used.

### (Carbon dioxide absorption aid 4)

(A) 1,3-Butylene glycol as an alcohol was dehydrated with calcium chloride, and then filtration was carried out with filter paper, thus producing a carbon dioxide absorption aid 4 (Example 7).
(B) Olive oil as oils and fats was dehydrated with calcium chloride, and then filtration was carried out with filter paper, thus producing a carbon dioxide absorption aid 4 (Example 8).
(C) Avocado butter, which is a kind of oils and fats, was used as a carbon dioxide absorption aid 4 (Example 9).
(D) An emulsion was prepared using 1 part by weight of cetyl alcohol, 2.5 parts by weight of whitepetrolatum and 6 parts by weight of squalane as oils and fats, 2 parts by weight of stearic acid, 1 part by weight of POE (20) hardened castor oil and 1 part by weight of glyceryl monostearate as surfactants, 36.9 parts by weight of distilled water as water, 0.1 part by weight of methylparaben as a preservative, and 4 parts by weight of glycerol, 0.5 part by weight of sodium carboxymethyl cellulose and 5 parts by weight of ethanol as other raw materials, and this was used as a carbon dioxide absorption aid 4 (Example 10).
(E) Jojoba oil, which is a kind of wax, was used as a carbon dioxide absorption aid 4 (Example 11).

The carbon dioxide absorption aids 4 defined in (A) to (E) above were applied thinly onto the back of the left hand of three females of ages 25, 37 and 40 years and a male of age 48 years, and with nothing applied to the back of the right hand, the hands were put respectively into the two sealing enclosure members 2a and 2b, and carbon dioxide was initially supplied into each of the sealing enclosure members 2 for 10 seconds at a rate of 200 mL/s from the aforementioned carbon dioxide supply means 3, and then for 7 minutes at a rate of 20 mL/s.

For all of the subjects, the skin temperature of both hands rose, but for everyone the skin on the back of the left hand felt warmer, and the skin on the back of the left hand reddened, and hence vasodilation was observed. On the other hand, for all of the subjects, the skin on the back of the right hand on which nothing had been applied was not red immediately after the carbon dioxide absorption aid 4 had been removed.

Immediately after the carbon dioxide absorption aid 4 was removed, for all of the subjects, the back of the left hand was whiter and smoother than the back of the right hand, and hence whitening and skin beautification effects were observed. Furthermore, the different combinations of the different absorption aids 4 out of the aforementioned (A) to (E) were applied onto the backs of the left and right hands of each subj ect, and carbon dioxide absorption was carried out as before, whereupon the whitening effect was strongest for (D).

### [Example 12]

### (Sealing enclosure member 2)

As shown in FIG. 6, a vinyl bottle of diameter 7 cm was cut sideways in a position 6 cm below the mouth, and thin sponge was glued around the circumference of the cut edge using double-sided tape. A thin rubber balloon (e.g. a condom is suitable) was placed over the mouth of the bottle, and the mouth part was bound with a rubber band. A hole was made in the side of the bottle, a plastic carbon dioxide injecting tube 2a of diameter 5 mm and length 3 cm was inserted in, and a tube 3a was connected thereto, thus producing a sealing enclosure member 2.

### (Carbon dioxide supply means 3)

Liquefied carbon dioxide gas filled into a high-pressure gas cylinder (filling amount: 30 kg) was used.

### (Carbon dioxide absorption aid 4)

A carbon dioxide absorption aid 4 of pH 2.5 was prepared using 50 parts by weight of a 1.0% aqueous sodium hyaluronate solution as a thickener, 1 part by weight of malic acid as an acid, and 49 parts by weight of purified water as water, and this was impregnated in an amount of 0.05 g per square centimeter into a cotton nonwoven cloth, thus producing a carbon dioxide absorption aid 4.

### (Evaluation test)

An 8 cm × 8 cm square of the carbon dioxide absorption aid 4 was patched onto the abdomen of a 48-year-old male, the sealing enclosure member 2 was placed thereover, the part contacting the skin was slightly raised up, and carbon dioxide was supplied in for 10 seconds at a rate of 200 mL/s, thus replacing the air in the sealing enclosure member 2 with carbon dioxide.

Next, the sealing enclosure member 2 was adhered to the skin, and carbon dioxide was supplied in until the balloon 5 expanded. The system was left for 5 minutes, whereupon the balloon deflated, and hence the sealing enclosure member 2 was removed from the abdomen, whereupon the skin under the absorption aid 4 had reddened in a disk shape matching the shape of the part of the skin adhered to the sealing enclosure member 2, and hence strong dilation of the blood vessels in the skin of the abdomen was observed. A few minutes after the absorption aid 4 had been taken off, the redness of the skin of the abdomen disappeared, but now this part turned white in a disk shape, and hence a whitening effect was observed.

### (Comparative test)

In the above evaluation test, gas-impermeable wrapping film was used instead of the carbon dioxide absorption aid 4, whereupon reddening occurred in a circular shape due to the pressure with which the sealing enclosure member 2 was pushed against the skin, but reddening of the skin in a disk shape as in the above evaluation test was not observed, and hence vasodilation was not observed. Similarly, a whitening effect was not observed either.

### [Example 13]

### (Sealing enclosure members 2)

Sealing enclosure members 2 as in Example 5 were used.

### (Carbon dioxide supply means 3)

Liquefied carbon dioxide gas filled into a high-pressure gas cylinder (filling amount: 30 kg) was used.

### (Carbon dioxide absorption aid 4)

A carbon dioxide-dissolving medium comprising a viscous material containing water was prepared using 1 part by weight of propylene glycol alginate and 1.2 parts by weight of sodium carboxymethyl cellulose as thickeners, 86.7 parts by weight of purified water as water, 0.1 part by weight of methylparaben as a preservative, and 1 part by weight of sodium dihydrogen phosphate as a pH adjuster, and 0.04 g per square centimeter was impregnated into a cotton nonwoven cloth, thus preparing a carbon dioxide absorption aid 4.

### (Evaluation test)

A 5 cm × 5cm square of the carbon dioxide absorption aid 4 was patched onto the back of the left hand of a 48-year-old male, and a cotton nonwoven cloth impregnated with 0.04 g per square centimeter of the carbon dioxide absorption aid 4 in Example 6 was patched onto the back of the right hand. The two hands were put respectively into the two sealing enclosure members 2a and 2b, and carbon dioxide was initially supplied into each of the sealing enclosure members 2 for 10 seconds at a rate of 200 mL/s from the aforementioned carbon dioxide supply means 3, and then for 7 minutes at a rate of 20 mL/s. Both hands of the male immediately became warm, and no difference was felt between the left and the right.

Immediately after ceasing the supplying in of the carbon dioxide, the respective absorption aids were removed, whereupon the redness was stronger for the skin on the back of the left hand than the skin on the back of the right hand, showing that the vasodilation effect was stronger. The two hands were subsequently washed, whereupon for the backs of both hands, the skin had become smooth and white at the part on which the absorption aid had been patched, and hence whitening and skin beautification effects were observed, but in both cases the back of the left hand was better than the right hand.

### INDUSTRIAL APPLICABILITY

The present invention relates to a carbon dioxide external administration device, and can be used for readily obtaining assured cosmetic and medical effects.

## Claims

1. A carbon dioxide external administration device, **characterized by** comprising:
a sealing enclosure member capable of sealing a body surface from the outside air;
a supply means for supplying carbon dioxide into the inside of the sealing enclosure member; and
an absorption aid that assists transdermal or transmucosal absorption of the carbon dioxide inside the sealing enclosure member.

2. The carbon dioxide external administration device according to claim 1, **characterized by** having a carbon dioxide amount indicator that expands upon carbon dioxide being supplied into the sealing enclosure member, and contracts by the decrease of carbon dioxide.

3. The carbon dioxide external administration device according to claim 1 or 2, **characterized in that** the carbon dioxide absorption aid contains at least one carbon dioxide-dissolving medium selected from the group consisting of water, alcohols having a high vaporization temperature, oils and fats, and waxes.

4. The carbon dioxide external administration device according to any of claims 1 through 3, **characterized in that** the carbon dioxide absorption aid is a sheet-like product impregnated with a liquid containing at least water.

5. The carbon dioxide external administration device according to any of claims 1 through 3, **characterized in that** the carbon dioxide absorption aid contains a carbon dioxide-dissolving medium comprising a viscous material containing at least water.

6. The carbon dioxide external administration device according to claim 5, **characterized in that** the carbon dioxide-dissolving medium comprising a viscous material containing at least water contains at least one thickener selected from the group consisting of sodium alginate, propylene glycol alginate, carrageenan, sodium hyaluronate, pectin, polyvinyl alcohol and polyvinyl pyrrolidone.

7. The carbon dioxide external administration device according to any of claims 1 through 5, **characterized in that** the carbon dioxide absorption aid contains a carbon dioxide-dissolving medium comprising at least a calcium alginate hydrogel.

8. The carbon dioxide external administration device according to any of claims 1 through 5, **characterized in that** the carbon dioxide absorption aid contains a carbon dioxide-dissolving medium that is an emulsion or cream comprising at least an oil or a fat, a surfactant and water.
